Europäisches Patentamt

European Patent Office

Office européen des brevets

(11) Veröffentlichungsnummer : **0 135 728 B1**

## (12) EUROPÄISCHE PATENTSCHRIFT

(45) Veröffentlichungstag der Patentschrift :
11.11.87

(21) Anmeldenummer : 84109154.9

(22) Anmeldetag : 02.08.84

(51) Int. Cl.⁴ : **C 07 C 51/47, C 07 C 59/08, C 07 C 59/265, C 08 F226/00**

(54) Isolierung von enzymatisch erzeugten Carbonsäuren.

(30) Priorität : 04.08.83 DE 3328093

(43) Veröffentlichungstag der Anmeldung :
03.04.85 Patentblatt 85/14

(45) Bekanntmachung des Hinweises auf die Patenterteilung : 11.11.87 Patentblatt 87/46

(84) Benannte Vertragsstaaten :
AT BE CH DE FR GB IT LI NL SE

(56) Entgegenhaltungen :
EP-A- 0 049 429
DE-A- 2 450 670
DE-A- 3 043 766

(73) Patentinhaber : HOECHST AKTIENGESELLSCHAFT
Postfach 80 03 20
D-6230 Frankfurt am Main 80 (DE)

(72) Erfinder : Keil, Karl-Heinz, Dr.
Lübecker Weg 3
D-6450 Hanau (DE)
Erfinder : Greiner, Ulrich, Dr.
Nidderauer Strasse 13
D-6369 Schöneck (DE)
Erfinder : Engelhardt, Friedrich, Dr.
Hünfeldstrasse 41
D-6000 Frankfurt am Main 61 (DE)
Erfinder : Kühlein, Klaus, Dr.
Fasanenstrasse 41
D-6233 Kelkheim (Taunus) (DE)
Erfinder : Hess, Gerhard
Düdelsheimer Strasse 7
D-6472 Altenstadt (DE)
Erfinder : Keller, Reinhold, Dr.
Wiesenweg 5
D-6232 Bad Soden am Taunus (DE)
Erfinder : Schlingmann, Merten, Dr.
Schneidhainer Strasse 32a
D-6240 Königstein/Taunus (DE)

## Beschreibung

Die Isolierung von Carbonsäuren, die nicht oder nur schwer durch Destillation abgetrennt werden können, ist recht aufwendig. Enzymatisch, z. B. fermentativ erzeugte Hydroxycarbonsäuren wie Milchsäure und Zitronensäure werden deshalb häufig in Form der Calciumsalze ausgefällt, aus denen dann mit Schwefelsäure die Carbonsäuren wieder freigesetzt werden. Hierbei wird die bis zu 25fache Gewichtsmenge an feuchtem Gips erhalten, der im allgemeinen nicht unter vertretbaren Kosten aufgearbeitet werden kann und deshalb deponiert werden muß.

Im folgenden soll unter den Begriffen « Fermentation » bzw. « fermentativ » jede enzymatische Reaktion verstanden werden, also auch solche mit freien oder gebundenen Enzymen und mit toten Zellen.

Es ist bekannt, daß man fermentativ erzeugte Milchsäure mit Hilfe eines Anionenaustauschers isolieren kann, wobei die im Ionenaustauscher gebundene Milchsäure dann mit einer Calciumhydroxidlösung oder mit starker Schwefelsäure isoliert wird. Im ersteren Fall muß die Milchsäure aus dem Calciumlactat wieder freigesetzt werden und im letzteren Fall wird der Austauscher mit Sulfationen beladen, was zu einem Salzanfall fuhrt. Ein Nachteil beim Einsatz von Ionenaustauschern ist auch, daß andere Anionen aus der Fermentationslösung gebunden werden, also auch die der Nährsalze. Hierdurch wird nicht ñur die Kapazität des Ionenaustauschers beansprucht und die Aufarbeitung verteuert, sondern auch praktisch verhindert, daß Carbonsäuren kontinuierlich aus Fermentationslösungen abgetrennt werden und die restliche Lösung mit den darin enthaltenen Salzen wieder in die Fermentation zurückgeführt wird.

Es wurde nun gefunden, daß Carbonsäuren aus Fermentationsbrühen besonders vorteilhaft abgetrennt werden können, wenn man diese Fermentationsbrühen mit Polymerisaten behandelt, die tertiäre Aminogruppen enthalten, und die adsorbierten Carbonsäuren durch Behandeln mit einem polaren Lösemittel aus der Gruppe der aliphatischen Alkohole, aliphatischen Ketone und aliphatischen Carbonsäureester desorbiert.

Bevorzugte Ausgestaltungen dieser Erfindung werden im folgenden näher erläutert :

Geeignete Polymerisate mit tertiären Aminogruppen, die selektiv Carbonsäuren adsorbieren und deren Desorption mit den genannten polaren Lösemitteln erlauben, sind beispielsweise aus den deutschen Patentschriften 1 274 128 und 3 043 766 bekannt. Besonders vorteilhaft sind die in den deutschen Patentanmeldungen P 33 24 834.6 und P 33 24 835.4 vorgeschlagenen vernetzten Copolymerisate.

Aus der deutschen Patentschrift 3 043 766 ist es bekannt, Carbonsäuren aus technischen Lösungen bzw. Abwässern wiederzugewinnen. Es findet sich darin jedoch kein Anhaltspunkt dafür, daß dieses Verfahren auch für die Isolierung aus Fermentationslösungen geeignet ist, da diese Lösungen eine Vielzahl von organischen und anorganischen Komponenten gelöst bzw. suspendiert enthalten.

Eine vorteilhafte Ausgestaltung der Erfindung bezieht sich auf eine kontinuierliche Reaktion, wobei die aus dem Reaktor abfließende Lösung oder Suspension kontinuierlich durch einen von mehreren parallel geschalteten Desorbern läuft, wobei die aus dem Desorber austretende Flüssigkeit — gegebenenfalls nach Aufstärken mit Nährstoffen und/oder Nährsalzen — in den Reaktor zurückgeführt wird. Nach Beladen eines Desorbers wird die Reaktionslösung auf den nächsten Desorber geführt und aus dem beladenen Desorber die Carbonsäure mit Hilfe des polaren Lösemittels eluiert.

Bei dieser kontinuierlichen Ausgestaltung der Erfindung kann es vorteilhaft sein, nur einen mehr oder weniger großen Anteil der enthaltenen Carbonsäure zu adsorbieren und die restlichen Anteile im Kreislauf zurückzuführen.

Man kann so beispielsweise ein unerwünscht starkes Konzentrationsgefälle an Produkt zwischen FermentEerein- und -auslauf vermeiden, beispielsweise bei produktkatalysierten Fermentationsreaktionen.

Bei Fermentationslösungen bzw. -suspensionen mit einem hohen Anteil an Fremdsubstanzen kann es auch technisch vorteilhaft sein, die Carbonsäuren nur zum Teil zu adsorbieren, um den apparativen Aufwand gering zu halten. Bei einem Kreislaufverfahren spielt dann der im Kreisstrom verbleibende Anteil an Produkt praktisch keine Rolle.

Besonders vorteilhaft ist eine Ausgestaltung der Erfindung, bei der die Fermentation mit Hilfe von fixierten Biokatalysatoren erfolgt, wobei es sich bei diesen fixierten Katalysatoren um trägergebundene Enzyme oder fixierte Mikroorganismen handeln kann. Geeignete perlförmige Biokatalysatoren sind beispielsweise in den deutschen Offenlegungsschriften 2 343 633 und 2 805 607 beschrieben sowie in der deutschen Patentanmeldung P 32 37 341.4 vorgeschlagen. Besonders vorteilhaft werden solche fixierten Biokatalysatoren in einem Reaktor nach dem Schlaufenprinzip eingesetzt, wie es in der deutschen Patentanmeldung P 32 47 214.5 vorgeschlagen wurde.

Die Behandlung der Fermentationsbrühen mit dem Adsorptionsmittel erfolgt in üblicher Weise. Für kontinuierliche Verfahren kann das Adsorptionsmittel in Fließbett- oder Festbett-Reaktoren angeordnet sein.

Die Desorption der Carbonsäure erfolgt zweckmäßig mit leicht-flüchtigen polaren Lösemitteln wie Essigsäuremethyl- oder -ethylester, Azeton oder Methyl-ethyl-keton und insbesondere mit niederen Alkanolen wie Ethanol und besonders vorteilhaft Methanol.

Die Desorption der Carbonsäuren aus fest angeordneten Adsorptionsmittel kann im Gleich- oder Gegenstrom, bez. auf die Beladung, erfolgen.

Bevorzugt ist das Gegenstromverfahren.

Die Abtrennung der Carbonsäure aus dem Eluat erfolgt nach bekannten Methoden. Beim Einsatz leicht flüchtiger Elutionsmittel können diese leicht durch Destillation abgetrennt werden. Je nach der Löslichkeit des Produktes kann es vorteilhaft sein, nur einen Teil des Elutionsmittels abzudestillieren und das Produkt aus der konzentrierten Lösung zu kristallisieren.

Es ist selbstverständlich auch möglich, dem Elutionsmittel Basen, wie beispielsweise Ammoniak zuzusetzen, wobei dann jedoch — je nach Basenmenge anteilig — das entsprechende Salz erhalten wird. Wenn dieses Salz leicht zerlegbar, beispielsweise thermolabil, ist, kann auch auf diesem Wege die freie Carbonsäure erhalten werden. Gegebenenfalls kann auf die organische Komponente auch ganz verzichtet werden, wobei dann die wäßrige Basenlösung das polare Elutionsmittel darstellt.

In den folgenden Beispielen wird die Erfindung näher erläutert. Prozentangaben beziehen sich hierbei auf das Gewicht, sofern nichts anderes angegeben ist.

Beispiel 1

20 g abzentrifugierte Lactobacillus bulgaricus-Zellen werden nach Beispiel 1 der deutschen Patentanmeldung P 32 37 341.4 immobilisiert und in eine auf 42 °C thermostatisierte Säule mit 5,0 cm Innendurchmesser gefüllt. Aus einem 3 l-Vorratsgefäß wird eine 10 %ige Glucoselösung vom ·pH 6,5 durch die Säule gepumpt. Die Durchflußgeschwindigkeit wird dabei so eingestellt, daß der pH-Wert des Eluats 4,0 nicht unterschreitet.

Zur Adsorption der gebildeten D-Milchsäure wird das Eluat über eine zweite Säule gepumpt, welche mit 50 g des nach Beispiel 5 a) der deutschen Patentanmeldung P 33 24 834.6 hergestellten Adsorberharzes gefüllt ist. Die von der D-Milchsäure befreite Substratlösung fließt in das Vorratsgefäß zurück. Nach Erreichen der Kapazitätsauslastung des Adsorbers (68,0 g Milchsäure/100 g Adsorber) desorbiert man die adsorbierte D-Milchsäure durch Elution mit Methanol.

Nach Abdestillieren des Methanols wird D-Milchsäure in einer Reinheit von 99 % in Form eines farblosen Öls gewonnen.

Durch Einsatz einer weiteren Adsorbersäule kann über 12 Tage D-Milchsäure kontinuierlich fermentiert und isoliert werden.

Beispiel 2

30 g abzentrifugierte Aspergillus niger-Zellen werden gemäß Beispiel 1 der deutschen Patentanmeldung P 32 37 341.4 immobilisiert und in eine auf 30 °C thermostatisierte Säule mit 5,0 cm Innendurchmesser gefüllt. Aus einem 3 l-Vorratsgefäß wird eine 13 %ige Saccharoselösung vom pH 4,0 mit einer Durchflußgeschwindigkeit von einem Säulenvolumen pro Stunde über die Säule gepumpt. Zur Adsorption der gebildeten Zitronensäure wird das Eluat dieser Säule über eine zweite Säule gepumpt, die mit einem Adsorberharz gefüllt ist, das nach Beispiel 6 a der deutschen Patentanmeldung P 33 24 834.6 erhalten wurde. Das von Zitronensäure befreite Eluat fließt in das Vorratsgefäß zurück. Nach Erreichen der Kapazitätsauslastung des Adsorbers (95 g Zitronensäure/100 g Adsorber) wird die beladene Säule durch eine zweite Adsorbersäule ersetzt. Die Desorption der Zitronensäure erfolgt durch Elution mit Methanol. Nach Aufkonzentrieren der methanolischen Lösung und Abkühlen auf 5 °C erhält man 85 g Zitronensäure in Form farbloser Kristalle.

Beispiel 3

20 g abzentrifugierte Lactobacillus delbrueckii-Zellen werden in 20 g 5 %iger Natriumalginatlösung suspendiert und bei 20 °C durch eine Kanüle von 1,0 mm Durchmesser in ein Vernetzerbad, bestehend aus 2 l einer 0,5 molaren Calciumchloridlösung, getropft und 20 Minuten gerührt. Die entstandenen Kügelchen mit einem mittleren Radius von 2 mm werden abfiltriert und in 2 l einer Substratlösung, enthaltend 10 Gew.-% Glucose und 0,1 Gew.-% Hefeextrakt, gegeben. Die Lösung wird auf 42 °C thermostatisiert und gerührt. Durch kontinuierliche Zugabe von 5 N-Natronlauge über ein Autotitratorgerät wird der pH-Wert der Lösung auf 6,2 gehalten. Nach vollständigem Verbrauch der Glucose werden die Biokatalysator-Kügelchen abfiltriert. Die filtrierte Lösung wird mit konzentrierter Salzsäure auf pH 2,0 gestellt und zur Adsorption der gebildeten D-Milchsäure über eine Säule gepumpt, die mit 300 g dem nach Beispiel 13a der deutschen Patentanmeldung P 33 24 835.4 erhaltenen Harz gefüllt ist. Nach erfolgter Adsorption wird das Adsorberharz mit 2 Bettvolumina entionisiertem Wasser gewaschen und anschließend durch Elution mit Methanol die D-Milchsäure desorbiert. Nach Abdestillierten des Methanols erhält man 178 g D-Milchsäure in Form eines farblosen Öls.

Beispiel 4

2 l einer autoklavierten Nährlösung, enthaltend pro Liter 140 g Saccharose, 2,23 g Ammoniumnitrat, 1,0 g Dikaliumphosphat und 0,23 g Magnesiumsulfat-Heptahydrat, werden mit konzentrierter Salzsäure auf pH 2,0 eingestellt und mit Aspergillus niger angeimpft. Die Kultur wird bei 30 °C leicht geschüttelt. Nach 5 Tagen beträgt die Zitronensäurekonzentration 27 g/l (enzymatisch ermittelt). Die Kulturlösung wird von der Zellmasse abfiltriert und das Filtrat über eine Säule, gefüllt mit 70 g des nach Beispiel 5a der deutschen Patentanmeldung P 33 24 835.4 erhaltenen Harzes, gepumpt. Danach wird das Adsorberharz mit 2 Bettvolumina entionisiertem Wasser gewaschen und die adsorbierte Zitronensäure mit Methanol eluiert. Das Methanol wird zum größten Teil abgedampft, wobei 48 g Zitronensäure in Form farbloser Kristalle anfallen.

Anhang :
Beispiele 5a und 6a aus der deutschen Patentanmeldung P 33 24 834.6 :

Beispiel 5

a) 55 g 4-Vinyl-pyridin, 5 g 1,7-Bis-(3-hydroxy-
propyl)-1,1,3,3,5,5,7,7-octamethyl-tetrasiloxan-bis-
acrylat werden zusammen mit 0,22 g Dibenzopylperoxid gelöst und unter ständigem Durchleiten
von Stickstoff und unter ständigem Rühren in
eine Lösung aus 300 ml Wasser 37,5 g Natriumformiat und 37,5 g einer 1 %igen wäßrigen Hydroxyethylcellulose-Lösung gegeben. Dann wird auf
70 °C angeheizt und 1 h polymerisiert. Das erhitztene Copolymerisat wird mit Wasser gewaschen,
dann anschließend mit Methanol digeriert und
getrocknet.
Die Ausbeute beträgt 48 g.

Beispiel 6

a) 55 g 4-Vinyl-pyridin, 5 g Borsäure-triallylester
werden zusammen mit 0,22 g Dibenzoyl-peroxid
gelöst und unter ständigem Durchleiten von Stickstoff und unter ständigem Rühren in eine Lösung
aus 300 ml Wasser 37,5 g Natriumformiat und
37,5 g einer 1%igen wäßrigen Hydroxyethylcellulose-Lösung gegeben. Dann wird auf 60 °C erhitzt
und 1 h polymerisiert. Das erhaltene perlförmige
Copolymerisat wird abgetrennt mit Methanol gewaschen und getrocknet. Die Ausbeute beträgt
52 g.
Beispiele 5a und 13a aus der deutschen Patentanmeldung P 33 24 835.4 :

Beispiel 5

a) 120 g 1-Vinyl-imidazol, 40 g N-Vinylmethylacetamid und 20 g N,N'-Methylen-bis-acrylamid
werden bei Raumtemperatur mit 180 ml Wasser
und 1 g 4,4'-Azocyanopentansäure verrührt. Diese
Lösung wird in 1 200 ml n-Heptan, das 4 g lipophiles Schutzkolloid enthält, eingerührt. Unter ständigem Rühren und Durchleiten von Stickstoff
wird auf 65 bis 70 °C erwärmt und 2 h copolymerisiert. Das erhaltene perlförmige Copolymerisat
wird abgetrennt, gewaschen und getrocknet.
Die Ausbeute beträgt 162 g.

Beispiel 13

a) 40 g 1-Vinyl-imidazol, 2,5 g N,N'-Methylen-
bis-acrylamid und 2,5 g 1,7-Bis-(2-hydroxypropyl)-1,1,3,3,5,5,7,7-octamethyl-tetrasiloxan-bisac-
rylat werden zusammen mit 4,4'-Azobiscyanopentansäure und 45 ml Wasser gelöst und in 300 ml
n-Heptan und 1 g lipophilem Schutzkolloid eingerührt. Unter ständigem Rühren und Durchleiten
von Stickstoff wird auf 70 °C geheizt und 1 h
copolymerisiert. Das entstandene hochvernetzte,
strukturierte perlförmige Copolymerisat wird abgetrennt, mit Aceton gewaschen und getrocknet.
Die Ausbeute beträgt 42 g.

## Patentansprüche

1. Verfahren zur Isolierung von Carbonsäuren
aus Fermentationsbrühen, dadurch gekennzeichnet, daß man Fermentationsbrühen mit Polymerisaten behandelt, die tertiäre Aminogruppen enthalten, und die adsorbierten Carbonsäuren durch
Behandeln mit einem polaren Lösemittel aus der
Gruppe der aliphatischen Alkohole, aliphatischen
Ketone und aliphatischen Carbonsäureester desorbiert.

2. Verfahren nach Anspruch 1, dadurch gekennzeichnet, daß die aus einer kontinuierlichen
Reaktion aus dem Reaktor abfließende Lösung
oder Suspension kontinuierlich durch einen von
mehreren parallelgeschalteten Desorbern läuft
und die austretende Flüssigkeit in den Reaktor
zurückgeführt wird.

3. Verfahren nach Anspruch 2, dadurch gekennzeichnet, daß nur ein Anteil der enthaltenen
Carbonsäuren adsorbiert wird.

4. Verfahren nach Anspruch 1-3, dadurch gekennzeichnet, daß das Adsorptionsmittel in einem
Festbett-Reaktor angeordnet ist.

5. Verfahren nach Anspruch 4, dadurch gekennzeichnet, daß die Desorption im Gegenstrom
zur Beladungsrichtung erfolgt.

6. Verfahren nach einem oder mehreren der
vorhergehenden Ansprüche, dadurch gekennzeichnet, daß das Desorptionsmittel leicht flüchtig
ist.

7. Verfahren nach Anspruch 6, dadurch gekennzeichnet, daß das Desorptionsmittel Essigsäuremethyl- oder -ethylester, Azeton, Methyl-ethyl-
keton, Ethanol oder Methanol ist.

8. Verfahren nach Anspruch 6 oder 7, dadurch
gekennzeichnet, daß das Elutionsmittel ganz oder
teilweise von der Carbonsäure abdestilliert wird.

9. Abänderung des Verfahrens nach Anspruch
1, dadurch gekennzeichnet, daß das polare Lösemittel ganz oder teilweise durch eine Base ersetzt
wird.

10. Verfahren nach Anspruch 9, dadurch gekennzeichnet, daß eine Base eingesetzt wird, die
zu einem leicht zerlegbaren Salz führt.

## Claims

1. A process for isolating enzymatically produced carboxylic acids from fermentation broths
wherein the fermentation broths are treated with
polymers which contain tertiary amino groups
and the adsorbed carboxylic acids are desorbed
by treatment with a polar solvent from the group
of the aliphatic alcohols, aliphatic ketones and
aliphatic carboxylic acid esters.

2. The process as claimed in claim 1, wherein
the solution or suspension produced in a continuous reaction, and leaving the reactor passes
continuously through one of a plurality of desorbers connected in parallel, and the issuing liquid
is recycled to the reactor.

3. The process as claimed in claim 2, wherein only a proportion of the carboxylic acids present is adsorbed.

4. The process as claimed in claims 1-3, wherein the adsorbent is arranged in a fixed bed reactor.

5. The process as claimed in claim 4, wherein the desorption is carried out in counter-current to the loading direction.

6. The process as claimed in one or more of the preceding claims, wherein the desorbing agent is readily volatile.

7. The process as claimed in claim 6, wherein the desorbing agent is methyl acetate, ethyl acetate, acetone, methyl ethyl ketone, ethanol or methanol.

8. The process as claimed in claim 6 or 7, wherein the eluant is wholly or partially removed from the carboxylic acid by distillation.

9. The process as claimed in claim 1, modified in that the polar solvent is wholly or partially replaced by a base.

10. The process as claimed in claim 9, wherein a base which gives an easily decomposable salt is employed.

### Revendications

1. Procédé d'isolement d'acides carboxyliques à partir de bouillons de fermentation, caractérisé en ce que l'on traite les bouillons de fermentation par des polymères qui comportent des groupes amino-tertiaires et en ce que l'on désorbe les acides carboxyliques adsorbés par traitement avec un solvant polaire du groupe des alcools aliphatiques, des cétones aliphatiques et des esters d'acides carboxyliques aliphatiques.

2. Procédé selon la revendication 1 caractérisé en ce que la solution ou la suspension, provenant d'une réaction continue, qui s'écoule du réacteur, circule en continu à travers un parmi plusieurs désorbeurs montés en parallèle et en ce que le liquide sortant est renvoyé dans le réacteur.

3. Procédé selon la revendication 2 caractérisé en ce que seule une portion des acides carboxyliques contenus est adsorbée.

4. Procédé selon les revendications 1 à 3 caractérisé en ce que l'adsorbant est disposé dans un réacteur à lit fixe.

5. Procédé selon la revendication 4 caractérisé en ce que la désorption se fait à contre-courant de la direction de charge.

6. Procédé selon l'une ou plusieurs des revendications précédentes caractérisé en ce que l'agent de désorption est facilement volatil.

7. Procédé selon la revendication 6 caractérisé en ce que l'agent de désorption est l'acétate de méthyle ou d'éthyle, l'acétone, la méthyléthylcétone, l'éthanol ou le méthanol.

8. Procédé selon la revendication 6 ou 7 caractérisé en ce que l'éluant est séparé totalement ou partiellement de l'acide carboxylique par distillation.

9. Modification du procédé suivant la revendication 1 caractérisée en ce que le solvant polaire est totalement ou partiellement remplacé par une base.

10. Procédé selon la revendication 9 caractérisé en ce que l'on emploie une base qui conduit à un sel facilement décomposable.